# EUROPEAN PATENT APPLICATION

(11) **EP 3 646 914 A1**
(43) Date of publication of application: **06.05.2020**
(21) Application number: 17916181.5
(22) Date of filing: 29.06.2017
(51) Int. Cl.: A61M 25/00

(54) **CATHETER**

(71) Applicant: Asahi Intecc Co., Ltd., Seto-shi, Aichi 489-0071 (JP)
(72) Inventor: MORIMOTO, Toshio, Seto-shi, Aichi 489-0071 (JP)
(74) Representative: Winter, Brandl, Fürniss, Hübner, Röss, Kaiser, Polte - Partnerschaft mbB
(86) International application number: PCT/JP2017/024028
(87) International publication number: WO 2019/003398

(57) **Abstract**

To provide a catheter with a catheter shaft including a coil body as a reinforcing body, the catheter having excellent flexibility.

A catheter includes a catheter shaft having an inner layer, a coil body formed of a wire spirally wound around the outer periphery of the inner layer, and an outer layer covering the coil body. The coil body includes a joint part between adjacent wires, and a main body part other than the joint part, the joint part is positioned on a distal end side of the catheter shaft than the main body part, the joint part has a slit that is a notch penetrating in the radial direction, the slit has one end and the other end, and one end is on the distal end side of the joint part than the other end, and between the wires included in the joint part, all parts except a part overlapping the slit are jointed.

## Description

### Field

The present invention relates to a catheter including a coil body as a reinforcing body.

### Background

Conventionally, there is used a catheter including a catheter shaft having a coil body formed by winding a single metal element wire or multiple metal element wires as a reinforcing body. With the use of the coil body as a reinforcing body, it is possible to effectively prevent the lumen of the catheter from being closed, and to ensure the flexibility and torque transmission of the catheter.

Patent Literature 1 (Japanese Patent No. 5954748) discloses a configuration in which a portion (melting portion (32d)) where the element wires forming the coil body are fused and integrated mutually is provided at the distal end of a coil body (30d). It is described that this may prevent the end of the coil body from being scattered (see paragraphs [0036] and [0039] of Patent Literature 1).

### Citation List

### Patent Literature

Patent Literature 1: Japanese Patent No. 5954748

### Summary

### Technical Problem

The catheter is required to have high flexibility in order to secure preferable passing performance through a bent lesion, a hard lesion, and the like and preferable following property to a tortuous blood vessel such as a peripheral blood vessel. In view of the above-described aspects, the present invention aims at providing a catheter with a catheter shaft including a coil body as a reinforcing body, the catheter having excellent flexibility.

### Solution to Problem

The inventor of the present invention has intensively studied to solve the above-described problems, and as a result, has arrived at each aspect of the present invention as follows.

That is, the catheter according to the first aspect of the present invention is a catheter including a catheter shaft having an inner layer, a coil body formed of a wire wound spirally around an outer periphery of the inner layer, and an outer layer covering the coil body, in which the coil body includes a joint part between adjacent wires, and a main body part other than the joint part, the joint part is positioned on a distal end side of the catheter shaft closer than the main body part is, the joint part has a slit that is a notch passing through the joint part in a radial direction, the slit has one end and the other end, and one end is located on the distal end side of the joint closer than the other end is, and between the wires included in the joint part, all parts except a part overlapping the slit are jointed.

### Summary

The catheter having the above-described configuration includes the joint part in which the wires are jointed to the distal end portion of the coil body included in the catheter shaft, which prevents the end portion of the coil body from being scattered. Moreover, with the slit that is a notch passing through the joint part in the radial direction, the resin material forming the outer layer can be introduced into the slit. In such a case, when the external force generating a bending stress is applied to the joint part, the joint part is deformed so that the resin material in the slit is deformed, thereby preventing an increase in bending stress of the joint part. That is, when the external force generating a bending stress is applied, the joint part is easily deformed by deformation of the resin material in the slit, and has smaller bending rigidity than the case without a slit. As a result, even when the joint part reaches a bent lesion, a hard lesion, a tortuous blood vessel, or the like, it is possible to secure preferably passing performance and following property of the portion of the catheter in the vicinity of the joint part.

According to the second aspect of the present invention, in the above-described catheter, the joint part includes portions facing each other in a circumferential direction with the slit interposed therebetween. In such a configuration, when the external force generating a bending stress is applied to the joint part, the joint part is deformed so that the portions facing each other in the circumferential direction with the slit interposed therebetween approach each other or are separated or displaced from each other.

Further, according to the third aspect of the present invention, in the above-described catheter, the slit is formed along a direction of an axis of the joint part, inclined with respect to the axis, or spirally around the axis. In such a configuration, when the external force generating a bending stress is applied to the joint part, the joint part can be deformed so that the slit is deformed.

According to the fourth aspect of the present invention, in the above-described catheter, the inner layer and the outer layer are joined to each other through the slit. In this manner, the distal end portions of the inner layer and the outer layer separated by the joint part are jointed through the slit, which improves the joint strength between the inner layer and the outer layer. Therefore, it is possible to reduce the possibility that the distal end of the outer layer is peeled off from the inner layer.

According to the fifth aspect of the present invention, in the above-described catheter, the slit extends to a boundary between the joint part and the main body part. In such a configuration, it is possible to reduce rigidity of the joint part in the vicinity of the boundary with the main body part, and thus prevent a sudden change of rigidity of the coil body at the boundary between the main body part and the joint part and prevent stress concentration on the boundary between the main body part and the joint part of the coil body. As a result, it is possible to reduce the possibility that the boundary between the main body part and the joint part of the coil body is broken.

According to the sixth aspect of the present invention, in the above-described catheter, the slit increases in width toward the boundary. In such a configuration, it is possible to further reduce the rigidity of the joint part toward the boundary with the main body part. Thus, it is possible to exert the effects of the fifth aspect more preferably.

Moreover, according to the seventh aspect of the present invention, the above-described catheter further includes a distal tip jointed to the distal end of the joint part, and the slit extends to the distal end of the joint part, and a part of a material forming the distal tip extends into the slit. In such a configuration, the part of the distal tip extending into the slit serves as an anchor, which improves the joint strength between the distal tip and the joint part.

The catheter according to the eighth aspect of the present invention is a catheter including a catheter shaft having an inner layer, a coil body formed of a wire wound spirally around an outer periphery of the inner layer, and an outer layer covering the coil body, in which the coil body includes a joint part between adjacent wires, and a main body part other than the joint part, the joint part includes an easily deformable portion, the easily deformable portion has a notch as a discontinuous portion that is discontinuous in the circumferential direction, the discontinuous part in the easily deformable portion is continuous from one end to the other end in the axial direction of the easily deformable portion, and between the wires included in the joint part, all parts except a part overlapping the discontinuous part are jointed. In such a configuration, it is possible to exert the same effects as the first aspect.

### Brief Description of Drawings

FIG. 1A is an overall view of a catheter according to a first embodiment of the present invention, and FIG. 1B is an enlarged view of a distal end portion of the catheter surrounded by a one-dot chain line IB in FIG. 1A.
FIG. 2 is a longitudinal sectional view of the distal end portion of the catheter illustrated in FIG. IB.
FIG. 3 is a front view illustrating a distal end portion of a coil body included in the catheter of FIG. 2.
FIG. 4 is a cross-sectional view taken along the line IV-IV of the distal end portion of the catheter illustrated in FIG. IB.
FIG. 5 is a cross-sectional view taken along the line V-V of the distal end portion of the catheter illustrated in FIG. 2.
FIG. 6 is a transverse sectional view of a distal end portion of a catheter according to a modification of the first embodiment.
FIG. 7 is a transverse sectional view of a distal end portion of a catheter according to another modification of the first embodiment.
FIG. 8 is a front view illustrating a distal end portion of a coil body included in a catheter according to a second embodiment of the present invention.
FIG. 9 is a transverse sectional view of a distal end portion of the catheter according to the second embodiment.
FIG. 10 is a front view illustrating a distal end portion of a coil body included in a catheter according to a third embodiment of the present invention.
FIG. 11 is a longitudinal sectional view of a distal end portion of the catheter according to the third embodiment.
FIG. 12 is a front view illustrating a distal end portion of a coil body included in a catheter according to a fourth embodiment of the present invention.
FIG. 13 is a front view illustrating a distal end portion of a coil body included in a catheter according to a modification of the fourth embodiment.

### Description of Embodiments

Hereinafter, there will be described a catheter 1 according to a plurality of embodiments and modifications embodying the present invention with reference to the enclosed drawings. The members that are common or similar in the embodiments and modifications are represented with the same or similar reference numerals.

### First Embodiment

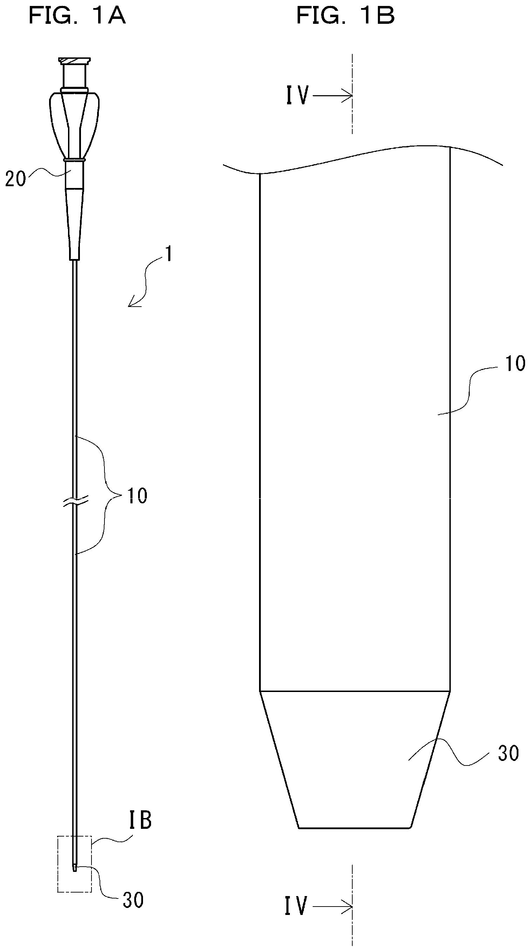

FIG. 1A is an overall view of the catheter 1 according to the first embodiment of the present invention, and FIG. 1B is an enlarged view of a distal end portion of the catheter 1 surrounded by a one-dot chain line IB in FIG. 1A. In FIGS. 1A and 1B, the lower side in the drawing is the distal end side (far side) inserted into a body, and the upper side is the rear end side (near side, proximal end side) operated by a technician such as a doctor.

The catheter 1 is used when treating a constricted section in a heart blood vessel, a bile duct, or a pancreatic duct, or when aspirating bone marrow in a bone, for example. As illustrated in FIGS. 1A and 1B, the catheter 1 according to this embodiment mainly includes a catheter shaft 10, a connector 20 attached to the rear end of the catheter shaft 10, and a distal tip 30 attached to the distal end of the catheter shaft 10.

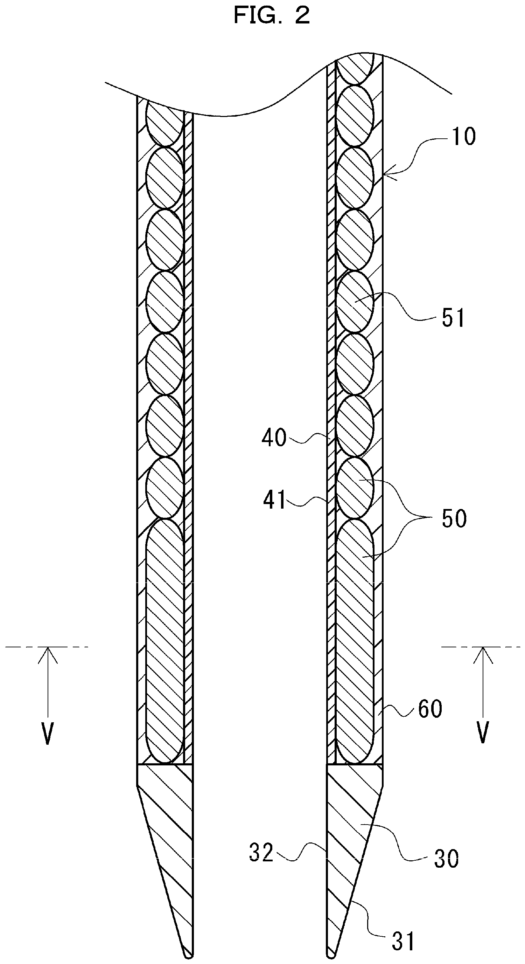

FIG. 2 is a longitudinal sectional view of the distal end portion of the catheter 1 illustrated in FIG. 1B, and is a cross section parallel to the paper surface of FIG. IB. As illustrated in FIG. 2, the catheter shaft 10 includes an inner layer 40 made of a resin material, a coil body 50 as a reinforcing body that is wound around the outer periphery of the inner layer 40, and an outer layer 60 made of a thermoplastic resin material that covers the outer periphery of the coil body 50.

The inner layer 40 forms a lumen 41 into which a guide wire or other catheters can be inserted. The resin material forming the inner layer 40 is not particularly limited. However, considering the slidability of a guide wire or other catheters inserted inside, PTFE (polytetrafluoroethylene) is preferable.

As a thermoplastic resin material forming the outer layer 60, it is possible to use polyamide, polyamide elastomer, polyester, polyurethane, or the like.

The distal tip 30 of the present embodiment includes an outer peripheral surface 31 that is reduced in diameter in a tapered shape toward the distal end, and a lumen 32 that communicates with the lumen 41 of the inner layer 40 and into which a guide wire or other catheters can be inserted. As the material of the distal chip 30, there is used a resin material that is more flexible than the resin material forming the inner layer 40 and the outer layer 60. For example, a polyurethane elastomer is used. The visibility of the distal tip 30 under X-ray fluoroscopy may be improved by mixing radiopaque powder such as tungsten powder in the distal tip 30.

In an example of the manufacturing process of the catheter 1, the coil body 50 is wound around the outer periphery of the inner layer 40, the outer periphery is further covered with thermoplastic resin that is a material of the outer layer 60, and the material of the outer layer 60 is heated and melted. Thereby, the outer layer 60 welded to cover the coil body 50 is formed. In addition to being welded to the coil body 50, the outer layer 60 also enters gaps between the coil bodies 50 and is also jointed to the inner layer 40. Further, the rear end surface of the distal tip 30 is brought into contact with the end surfaces of the inner layer 40 and the outer layer 60 and welded thereto.

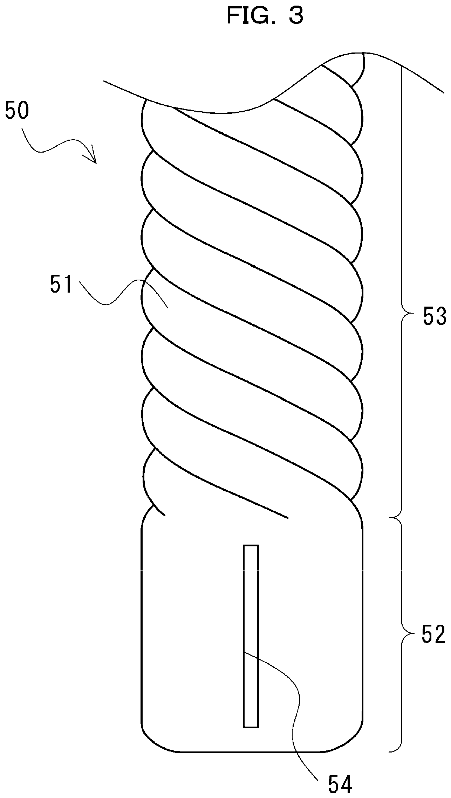

FIG. 3 is a front view illustrating a distal end portion of the coil body 50 taken out from FIG. 2. The coil body 50 is formed by spirally winding a plurality of wires 51 made of a metal material. The coil body 50 is not limited thereto, and it may be formed by spirally winding one wire 51 (single wire), or by spirally winding a stranded wire in which a plurality of single wires are twisted preliminarily as the wire 51. The metal material used for the wire 51 is not particularly limited, and it is possible to use stainless steel (SUS304, SUS316, etc.), gold, platinum, tungsten, titanium, nickel, or an alloy containing any of these elements.

In the catheter 1 of the present embodiment, at the distal end portion of the coil body 50, the wire 51 forming the coil body 50 is heated by a laser or the like and melted so that the wires 51 are jointed and integrated to form a joint part 52. This controls the behavior of the wires 51 at the distal end portion of the coil body 50. For example, when a hollow coil (a hollow body formed by spirally winding a plurality of wires) is used as the coil body 50, it is possible to effectively prevent the end of the hollow coil from being scattered (disengaged). A portion of the coil body 50 other than the joint part 52 is referred to as a main body part 53 of the coil body 50.

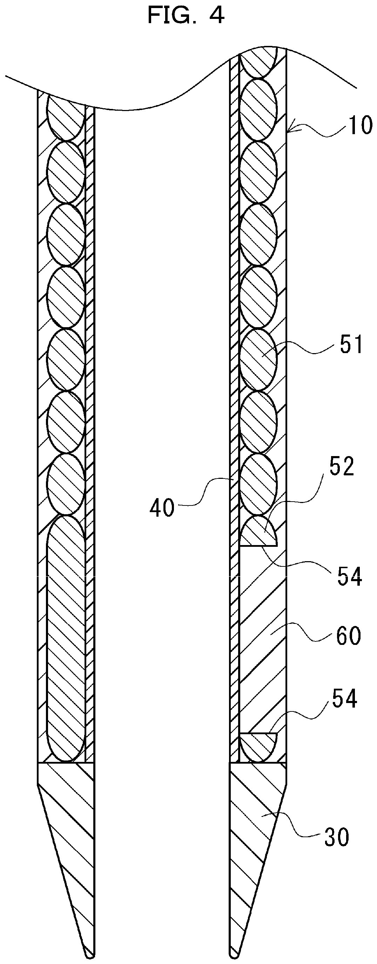

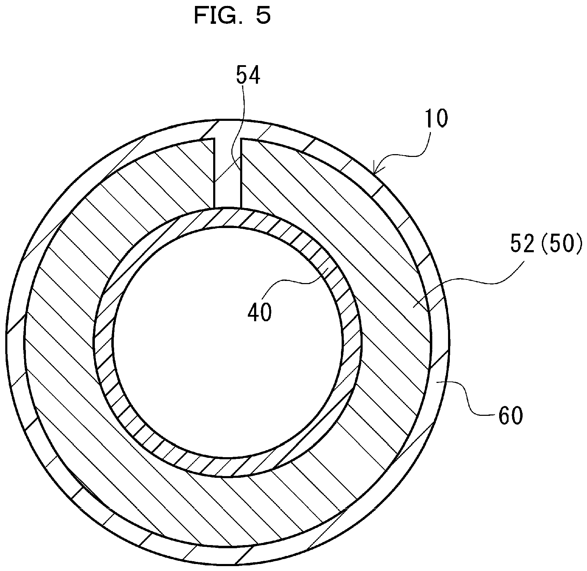

FIG. 4 is a cross-sectional view taken along the line IV-IV of the distal end portion of the catheter 1 of FIG. 1B, and FIG. 5 is a cross-sectional view taken along the line V-V of the catheter 1 of FIG. 2. As illustrated in FIGS. 3 to 5, the joint part 52 of the coil body 50 of the present embodiment has a slit 54 formed by cutting out the joint part 52 to penetrate in the radial direction using a laser, a diamond cutter or the like. The slit 54 of the present embodiment is formed in a linear shape along the direction of the axis of the joint part 52. Note that between the wires 51 included in the joint part 52, joint is maintained in all parts except the part overlapping the slit 54.

Such a slit 54 allows the deformation of the joint part 52. That is, the rigidity of the joint part 52 is lowered. The joint part 52 has the slit 54 as a discontinuous part discontinuous in the circumferential direction. Thus, such a range can be regarded as an easily deformable portion. In this easily deformable portion, the slit 54 that is a discontinuous part is continuous from one end to the other end in the axial direction of the easily deformable portion. When the external force generating a bending stress is applied to the joint part 52, the joint part 52 can be deformed so that the resin material of the outer layer 60 existing in the slit 54 is deformed. That is, when the external force generating a bending stress is applied, the joint part 52 is easily deformed by deformation of the resin material of the outer layer 60 existing in the slit 54, and has smaller bending rigidity than the case without a slit. This ensures the flexibility of the catheter 1 in the vicinity of the joint part 52. As a result, even when the joint part 52 reaches a bent lesion, a hard lesion, a tortuous blood vessel, or the like, it is possible to secure preferable passing performance and following property of the portion of the catheter 1 in the vicinity of the joint part 52. Further, even during the rotation operation of the catheter 1, it is possible to prevent stress concentration on the joint part 52, which reduces the possibility of breakage of the joint part. Therefore, the catheter 1 as a whole has high durability in addition to preferable passing performance and following property.

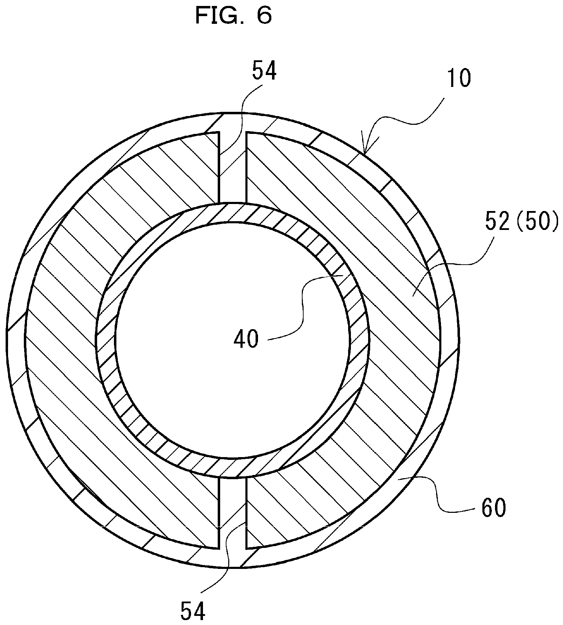

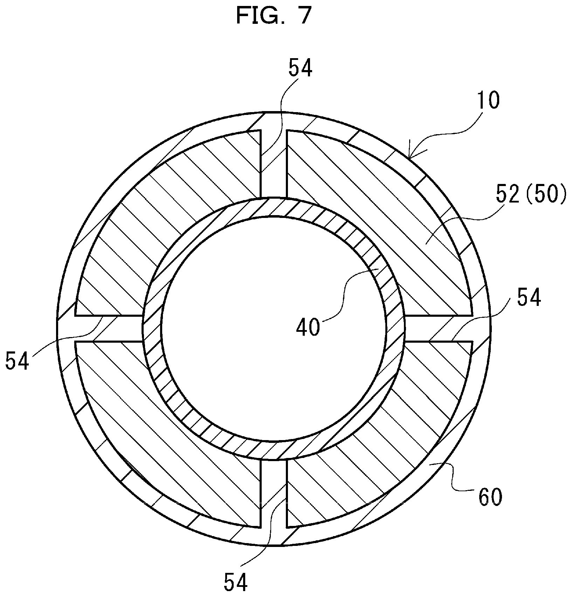

As illustrated in FIGS. 4 and 5, the inner layer 40 and the outer layer 60 are jointed to each other through the slit 54. In this manner, the distal end portions of the inner layer 40 and the outer layer 60 separated by the joint part 52 are jointed through the slit 54, which improves the joint strength between the inner layer 40 and the outer layer 60. Therefore, it is possible to reduce the possibility that the distal end of the outer layer 60 is peeled off from the inner layer 40. Furthermore, between the wires 51 included in the joint part 52, all parts except the part overlapping the slit 54 are jointed. Thus, even if the slit 54 is formed at the joint part 52, the strong joint is maintained between the wires 51 at the joint part 52. However, between the wires 51 included in the joint part 52, a part except the part overlapping the slit 54 may not be jointed partially. The number of slits 54 is not limited to that illustrated in FIGS. 3 to 5, and two or more slits 54 may be formed as illustrated in FIGS. 6 and 7. As illustrated in FIGS. 6 and 7, if the joint part 52 has portions facing each other in the circumferential direction with the slit 54 interposed therebetween, when the external force generating a bending stress is applied to the joint part 52, the joint part 52 can be deformed so that the portions facing each other in the circumferential direction with the slit 54 interposed therebetween approach each other, or are separated or displaced from each other. FIGS. 6 and 7 are transverse sectional views of the catheter 1 according to a plurality of modifications of the first embodiment, corresponding to FIG. 5 of the first embodiment.

### Second Embodiment

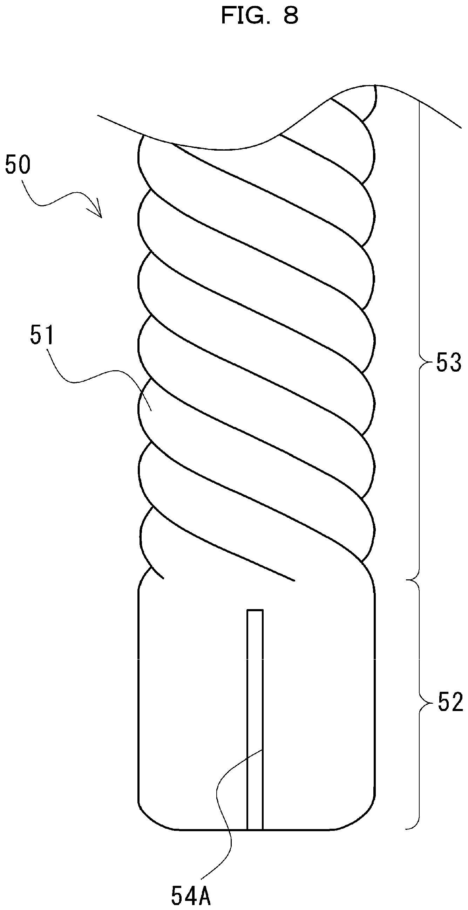

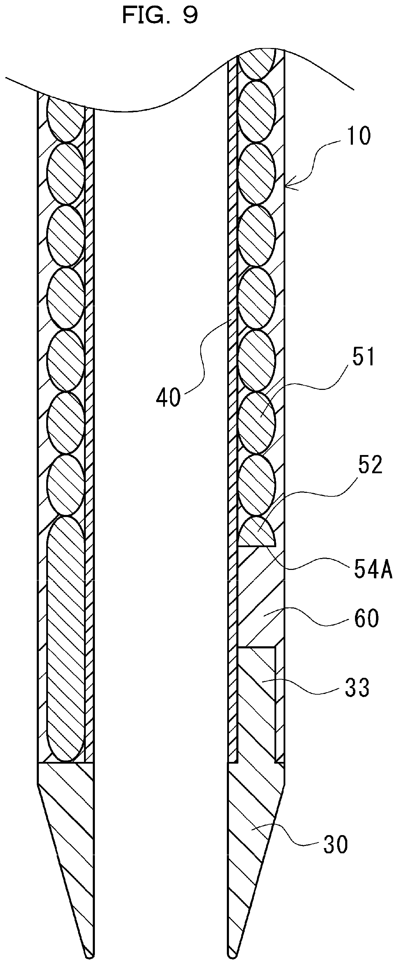

FIG. 8 is a front view illustrating the distal end portion of the coil body 50 included in the catheter 1 according to the second embodiment of the present invention, and FIG. 9 is a longitudinal sectional view of the distal end portion of the catheter 1 according to the second embodiment. FIGS. 8 and 9 correspond to FIGS. 3 and 4 of the first embodiment, respectively. In the present embodiment, the slit 54A formed in the joint part 52 of the coil body 50 extends to the distal end of the joint part 52, as illustrated in FIG. 8. Then, as illustrated by the reference numeral 33 in FIG. 9, a part of the material forming the distal tip 30 extends into the slit 54A. In such a configuration, the part 33 of the distal tip 30 introduced in the slit 54A serves as an anchor, which exerts the effect of further improving the joint strength between the distal tip 30 and the joint part 52, in addition to the effects of the above-described first embodiment. To exert the effects, the part 33 of the distal tip 30 introduced in the slit 54A is preferably welded to at least one of the inner layer 40, the coil body 50, and the outer layer 60.

As illustrated in FIG. 9, the part 33 in which the material of the distal tip 30 in introduced in the slit 54A extends to a halfway in the axis direction of the slit 54A and does not reach the end on the proximal end side of the slit 54A. In this manner, the presence of the portion 33 exerts the effects of improving the joint strength between the distal tip 30 and the joint part 52, and the inner layer 40 and the outer layer 60 are jointed to each other through the slit 54A, which also exerts the effects of improving the joint strength between the inner layer 40 and the outer layer 60. However, the part 33 may also reach the end on the proximal end side of the slit 54A.

### Third Embodiment

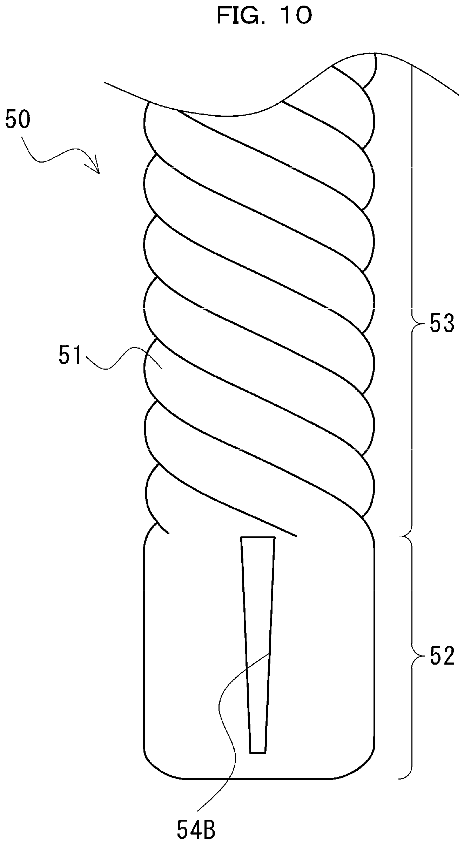

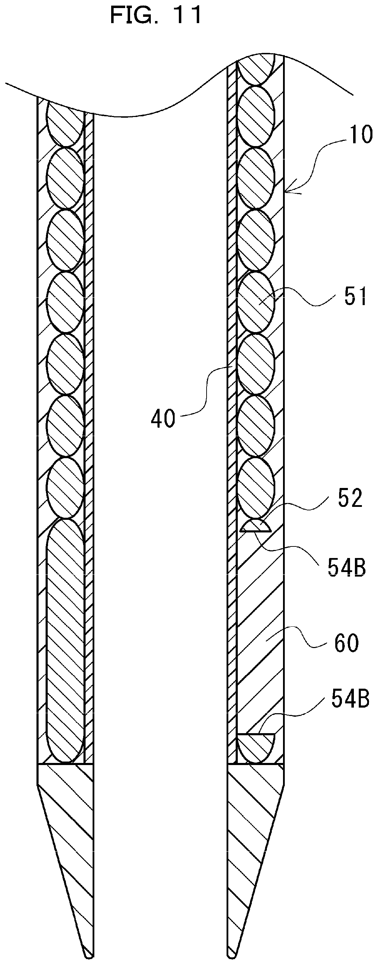

FIG. 10 is a front view illustrating the distal end portion of the coil body 50 included in the catheter 1 according to the third embodiment of the present invention, and FIG. 11 is a longitudinal sectional view illustrating the distal end portion of the catheter 1 according to the third embodiment. FIGS. 10 and 11 correspond to FIGS. 3 and 4 of the first embodiment, respectively. In the present embodiment, the slit 54B formed in the joint part 52 of the coil body 50 extends to the boundary between the joint part 52 and the main body part 53 of the coil body 50, as illustrated in FIG. 10. In such a configuration, it is possible to reduce rigidity of the joint part 52 in the vicinity of the boundary with the main body part 53, and thus prevent a sudden change of rigidity of the coil body 50 at the boundary between the main body part 53 and the joint part 52. In particular, as illustrated in FIG. 10, the slit 54B of the present embodiment expands in width toward the boundary between the joint part 52 and the main body part 53 of the coil body 50. In such a configuration, it is possible to effectively reduce rigidity of the joint part 52 in the vicinity of the boundary with the main body part 53, and thus improve the effects of preventing a sudden change of rigidity of the coil body 50 at the boundary between the main body part 53 and the joint part 52. As a result, it is possible to reduce the possibility that the boundary between the main body part 53 and the joint part 52 of the coil body 50 is broken.

### Fourth Embodiment

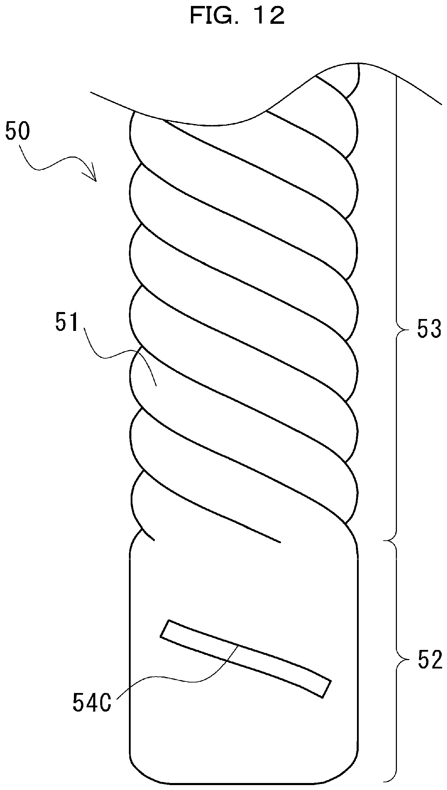

FIG. 12 is a front view illustrating the distal end portion of the coil body 50 included in the catheter 1 according to the fourth embodiment of the present invention. As in the present embodiment, the slit 54C formed in the joint part 52 of the coil body 50 may be formed inclined with respect to the axis of the joint part 52 or formed spirally around the axis. This also exerts the same effects as the first embodiment. In this case, the slit 54C may be formed along the wire 51 forming the joint part 52 or along the joint part between the wires 51.

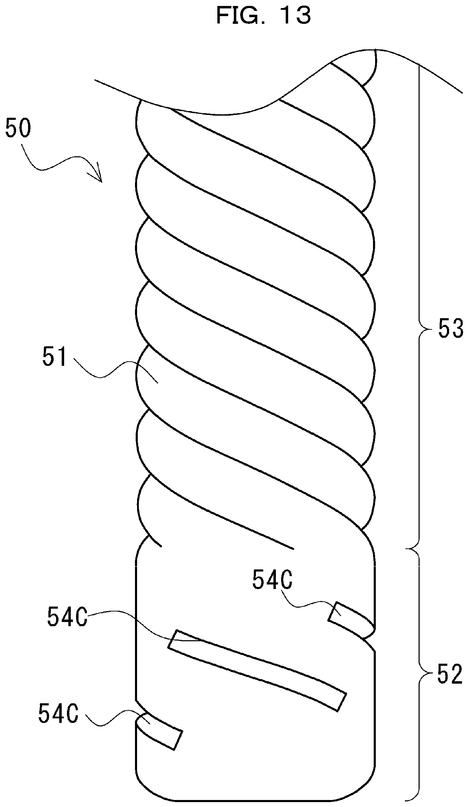

The number of slits 54C is not limited to one, and a plurality of slits 54C may be formed at positions displaced from each other in the axial direction and the circumferential direction of the joint part 52, as illustrated in FIG. 13, for example. In the case of the coil body 50 illustrated in FIG. 13, it can be understood that the joint part 52 is provided with a plurality of easily deformable portions respectively corresponding to the plurality of slits 54C so as to overlap in the axial direction.

The distal tip 30 is not necessarily formed of a resin material and may be formed of a metal material. The metal material in such a case is not particularly limited, and it is possible to use stainless steel (SUS304, SUS316, etc.), gold, platinum, tungsten, nickel, titanium, or an alloy containing any of these elements.

The catheter 1 according to the present invention may include a blade that is a braided body of metal wires arranged inside the coil body 50.

The above-described configurations included in the embodiments and modifications of the present invention may be implemented in combination with each other as long as they can be realized, and such combinations are also included in the present invention. For example, a slit formed in the joint part 52 of the coil body 50 may extend from the distal end of the joint part 52 to the boundary between the joint part 52 and the main body part 53.

### Reference Signs List

- 1: catheter
- 10: catheter shaft
- 20: connector
- 30: distal tip
- 40: inner layer
- 50: coil body
- 51: wire
- 52: joint part
- 53: main body part
- 54, 54A, 54B, 54C: slit
- 60: outer layer

## Claims

1. A catheter, comprising: a catheter shaft including an inner layer, a coil body that is formed of a wire spirally wound around an outer periphery of the inner layer, and an outer layer that covers the coil body, wherein
the coil body includes a joint part between adjacent wires, and a main body part other than the joint part,
the joint part is positioned on a distal end side of the catheter shaft closer than the main body part is,
the joint part has a slit that is a notch passing through the joint part in a radial direction,
the slit has one end and the other end, and the one end is located on the distal end side of the joint part closer than the other end is, and
between the wires included in the joint part, all parts except a part overlapping the slit are jointed.

2. The catheter according to claim 1, wherein the joint part includes portions facing each other in a circumferential direction with the slit interposed.

3. The catheter according to claim 1 or 2, wherein the slit is formed along a direction of an axis of the joint part, inclined with respect to the axis, or spirally around the axis.

4. The catheter according to any one of claims 1 to 3, wherein the inner layer and the outer layer are joined to each other through the slit.

5. The catheter according to any one of claims 1 to 4, wherein the slit extends to a boundary between the joint part and the main body part.

6. The catheter according to claim 5, wherein the slit increases in width toward the boundary.

7. The catheter according to any one of claims 1 to 6, further comprising: a distal tip that is jointed to the distal end of the joint part, wherein
the slit extends to the distal end of the joint part, and
a part of a material forming the distal tip extends into the slit.

8. A catheter, comprising: a catheter shaft including an inner layer, a coil body that is formed of a wire spirally wound around an outer periphery of the inner layer, and an outer layer that covers the coil body, wherein
the coil body includes a joint part between adjacent wires, and a main body part other than the joint part,
the joint part includes an easily deformable portion,
the easily deformable portion has a notch as a discontinuous part that is discontinuous in the circumferential direction,
the discontinuous part in the easily deformable portion is continuous from one end to the other end in the axial direction of the easily deformable portion, and
between the wires included in the joint part, all parts except a part overlapping the discontinuous part are jointed.
